Europäisches Patentamt

European Patent Office

Office européen des brevets

(19)

(11) Publication number: **0 196 864**
**A2**

(12) # EUROPEAN PATENT APPLICATION

(21) Application number: **86302201.8**

(22) Date of filing: **25.03.86**

(51) Int. Cl.⁴: **C 12 N 15/00**
**C 12 P 21/02**

(30) Priority: **25.03.85 US 715653**
**07.08.85 US 763932**

(43) Date of publication of application:
**08.10.86 Bulletin 86/41**

(84) Designated Contracting States:
**AT BE CH DE FR GB IT LI NL SE**

(71) Applicant: **CETUS CORPORATION**
**1400 Fifty-Third Street**
**Emeryville California 94608(US)**

(72) Inventor: **Chang, Shing**
**2289 Manzanita Drive**
**Oakland California 94611(US)**

(72) Inventor: **Lin, Leo Shun-Lee**
**1317 Chesterton Way**
**Walnut Creek California 94596(US)**

(72) Inventor: **Chang, Sheng-Yung**
**2289 Manzanita Drive**
**Oakland California 94611(US)**

(72) Inventor: **Wang, Alice Ming**
**2423 Providence Court**
**Walnut Creek California 94596(US)**

(74) Representative: **Bizley, Richard Edward et al,**
**BOULT, WADE & TENNANT 27 Furnival Street**
**London EC4A 1PQ(GB)**

(54) Alkaline phosphatase-mediated processing and secretion of recombinant proteins, DNA sequences for use therein and cells transformed using such sequences.

(57) Expression systems which are capable of secreting soluble, biologically active forms of proteins which are susceptible to processing in procaryotes under the influence of bacterial leader sequences is disclosed. Vectors successful in effecting this expression encode a fusion protein having an N-terminal sequence comprising the *phoA* (alkaline phosphatase) leader peptide and as a C-terminal sequence the desired protein. This fusion protein encoding sequence is placed under the control of a suitable bacterial promoter, preferably the alkaline phosphatase promoter. Terminator sequences may also be included in the vectors for efficient expression.

EP 0 196 864 A2

ALKALINE PHOSPHATASE-MEDIATED PROCESSING AND
SECRETION OF RECOMBINANT PROTEINS, DNA SEQUENCES
FOR USE THEREIN AND CELLS TRANSFORMED USING SUCH
SEQUENCES

The invention relates to the production of heterologous proteins in bacteria using recombinant techniques. More specifically, it relates to the use of alkaline phosphatase A gene (pho A) control and leader sequences to mediate processing and secretion of chimeric proteins resulting from alkaline phosphatase leader/heterologous protein fusions.

Recombinant production of chimeric or mature proteins containing polypeptides of desired amino acid sequence derived from heterologous sources is now well established. However, the resulting mature or chimeric proteins are, as a general rule, not secreted into the periplasmic space or into the medium, but rather accumulated intracellularly, often in the form of insoluble particles, variously known as inclusion or refractile bodies. These proteins are often troublesome to purify, at the minimum requiring that the cells be disrupted and separation be conducted from a plethora of intracellularly disposed protein materials, and, further, once purified may be deficient in biological activity, presumably due to inappropriate three dimensional confirmation. The severity of the problems associated with this form of recombinant production is variable but virtually always present to some degree when ATG-preceded "mature" coding sequences, or fusion proteins with, for example, N-terminal sequences of non-secreted bacterial polypeptides such as ß-galactosidase are placed downstream of bacterial promoters.

It was early realized that at least the purification problem with respect to recombinantly produced proteins could be simplified by effecting the secretion of the protein produced. U.S. Patents 4,411,994 and 4,338,397 to Gilbert, et al disclose the use of the penicillinase gene in pBR322 to produce fusion proteins presumably carried to the periplasmic space by the penicillinase leader sequence, using preproinsulin cDNA coding sequences inserted into the PstI site of pBR322. The resulting fusions (containing variable numbers of glycine residues between the penicillinase and insulin sequences due to the method of insertion) are indeed found in the periplasmic space of the transformed cells. Biological activity was not an issue, as the presence of the fusions was verified using immunological reactivity. The possibility of using the genes encoding other bacterial secreted proteins, including alkaline phosphatase A, is mentioned, but no description of how such utilization could be achieved is given.

Only a limited number of bacterial proteins are normally secreted. In E. coli, these proteins include penicillinase (when present), alkaline phosphatase, and various transport proteins. In Bacillus they further include α-amylase and subtilisin. These proteins, when produced natively are preceded by fused signal or leader peptides which are lost in the process of transporting the relevant protein across the cellular membrane. In gram-negative bacteria, such as E. coli, where the cytoplasmic membrane is encased in an outer cell membrane wall, the intervening volume being designated the "periplasmic space", this transport results in the presence of the protein in the periplasmic space. In gram-positive bacteria where the outer membrane is absent, however, the protein is secreted directly into the medium.

An attempt was made to utilize the alkaline phosphatase (phoA gene product) leader sequence to effect secretion of a foreign polypeptide by Ohsuye, K., et al, Nucleic Acids Res (1983) 11:1283. The coding sequence for α-neo-endorphin, a decapeptide hormone, was synthesized in vitro and ligated into vectors so as to produce fusion proteins of the endorphin with the major portion of the N-terminal sequence of alkaline phosphatase preceded by the phoA leader. The resulting chimeric proteins were successfully processed by removal of the signal, but were not transported to the periplasmic space. Again, biological activity of the resultant protein was not an issue.

It would be desirable to provide a system and method whereby recombinantly produced heterologous protein sequences are both secreted to ease purification and produced in biologically active form with proper folding. It appears from the results of the present invention that these goals are related. Accordingly, an expression system is required which results in processing and secretion of the heterologous protein produced thus providing a readily accessible source of correctly conformed material. The phoA constructions described below are capable of providing this result for susceptible heterologous sequences.

## Disclosure of the Invention

The invention provides a procaryotic expression system suitable for efficient production of desired heterologous proteins. The mature forms of susceptible heterologous polypeptide sequences are secreted through the cellular membrane in biologically active and soluble form and are thus readily purified. They are usable without the necessity for employing specialized procedures to correct conformational deficiencies attributable to host cell post-translation conditions. A pivotal feature of the expression system is the phoA leader sequence which is ligated into reading frame with the desired mature coding sequence.

Thus, in one aspect, the invention relates to an expression system for heterologous mature coding sequences which system comprises the phoA leader sequence upstream of, contiguous with, and in reading frame with the desired coding sequence. The expression system may also include the phoA promoter operably linked to the fused coding sequence and/or the phoA terminator downstream from and operably linked with the coding sequence. Other bacterial promoters and terminators or positive retroregulators may be substituted for the phoA controls. The expression system is typically disposed on a vector suitable to procaryotic hosts containing a compatible replicon and, desirably, a selectable marker.

The invention, in other aspects, includes such expression vectors and hosts transformed with them. The invention also relates to active, soluble, heterologous proteins produced by the expression system of the invention when resident in these hosts. The invention also relates to methods for producing such proteins, and to methods for improving the quality of recombinantly produced heterologous proteins.

Brief Description of the Drawings

Figure 1 shows the coding and amino acid sequence of the phoA leader altered to facilitate use in the expression system of the invention.

Figure 2 shows the construction of host vectors containing phoA leader and suitable control sequences.

Figure 3 shows the construction of expression vectors for interleukin-2 (IL-2) under control of the phoA promoter.

Figure 4 shows results of SDS-PAGE on extracts from E. coli transformed with an IL-2 expression vector.

Figure 5 shows the construction of expression vectors for human growth hormone (hGH).

Figure 6 shows the results of SDS-PAGE on extracts from E. coli transformed with an hGH expression vector.

Figure 7 shows the construction of expression vectors for human TNF.

Figure 8 shows the results of SDS-PAGE on extracts from E. coli transformed with a TNF expression vector.

Figure 9 shows the construction of an intermediate vector containing IL-2 encoding sequence.

Modes of Carrying Out the Invention

A. Definitions

As used herein, "susceptible protein" refers to a protein which has the appropriate amino acid sequence to permit interaction with bacterially derived phoA leader sequences to result in signal sequence processing and secretion of the susceptible protein. While the nature of the sequence related factor indigenous to the susceptible protein enabling this cooperation is not at present understood, it is clear that certain heterologous proteins are capable of interacting with the signal sequences derived from a variety of bacterial leaders, while others are incapable of doing so. Thus, proteins which are processed and secreted by phoA leader are also processed and secreted in processes mediated by amylase and penicillinase leaders. The converse is true, non-susceptible proteins are resistant and are found as fusion proteins with the pertinent leader sequence intracellularly. Examples of susceptible proteins include human growth hormone and TNF. Examples of non-susceptible proteins include IL-2.

"Secretion" refers to transport through the cytoplasmic membrane. Whether or not the protein appears in the medium is dependent on the presence or absence of an outer membrane; in the presence of outer membrane the secreted protein will be found in the periplasm, in the absence of outer membrane it will be in the medium.

"Alkaline phosphatase A gene" (phoA) refers to the alkaline phosphatase structural gene of E. coli K12 as, for example, disclosed by Kikuchi, Y., et al, Nucleic Acids Res (1981) 9:5671-5678. The structural gene is located at 8.5 minutes on the E. coli genetic map (Bachmann, B. J., et al, Microbiol Rev (1980) 44:1-56) and its native expression is relatively complex. However, the promoter and N-terminal regions have been sequenced (Kikuchi, Y., et al (supra)) and the sequence of the signal peptide deduced (Inouye, H., et al, J Bacteriol (1982) 149:434-439). The definition herein encompasses not only the specific structural gene and portions thereof, but functional equivalents derived from other bacterial sources or synthesized in vitro. It is understood that minor modifications may be made in the nucleotide sequences without affecting functionality, and that sequences derived from different strains or species of procaryotic cells may, and indeed almost surely do, contain sequences not absolutely identical to that of the above-mentioned source. In addition, in connection with the invention herein, modifications have been made to this sequence to provide suitable restriction cleavage sites, wherein these modifications do not result in loss of functionality.

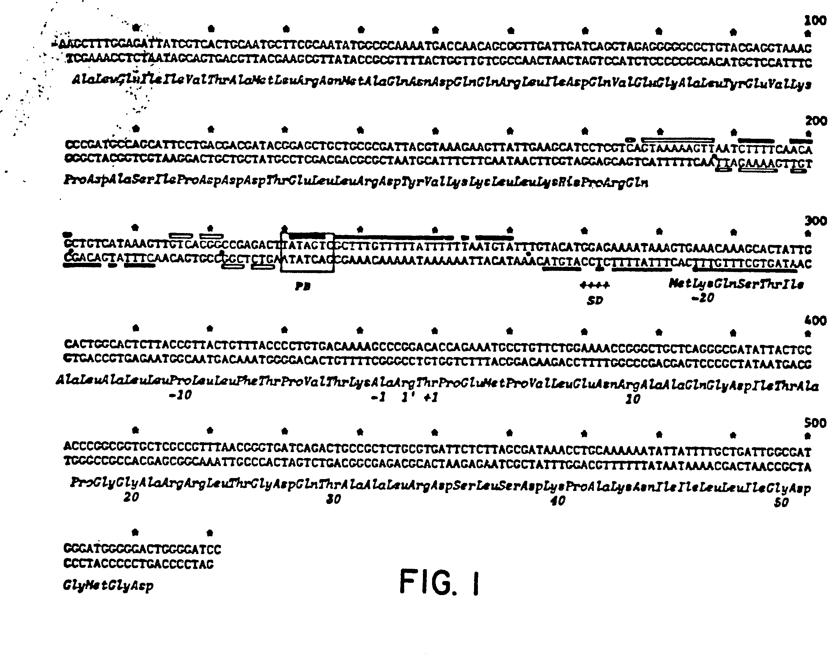

Of relevance to the present invention are the following regions of the alkaline phosphatase structural gene: the promoter, the ribosome binding site, the leader encoding sequence, and the terminator sequence. The nucleotide sequence of the 520 bp fragment which includes the promoter, ribosome binding site, and signal encoding are disclosed in Kikuchi, Y., (supra). The nucleotide sequence encoding the leader, modified to provide a NarI site is shown in Figure 1. This modification permits coding sequences other than that for alkaline phosphatase to be substituted in reading frame with leader, and in that sense the leader encoding sequence is still functional. However, conversion to the NarI site prevents processing of the preprotein with respect to alkaline phosphatase itself since the codon for the N-terminal arginine of the alkaline phosphatase sequence is thereby converted to that for proline. Functionality with respect to inserted sequences is not impaired as this portion of the NarI site is eliminated in the junctions.

"Operably linked" refers to juxtaposition wherein the functionality of the operably linked subjects are preserved. Thus, promoter operably linked to a coding sequence results in expression of the coding sequence under control of the promoter; desired protein operably linked to leader sequence refers to the protein disposed at the C-terminus of the leader. Positive retroregulator (conventionally referred to as terminator) operably linked to a coding sequence permits the positive retroregulator to enhance effective expression.

"Cells", "cell cultures", "host cells", "recombinant host cells" refer to subject cells for recombinant DNA manipulations. As would be apparent from the context, these cells may be candidates for, or resultants of, transfer of new DNA sequences according to recombinant techniques. Techniques which are suitable for DNA uptake by cells include, most prominently, in vitro transformation, however other techniques such as transduction or conjugation may also be used. The definition further includes the progeny of the cells directly referred to. It is understood that such progeny may not be precisely identical in DNA content to their parents, but such progeny are included in the definition so long as alterations due, for example, to accidental or deliberate mutation do not destroy the ability of the cells to exhibit the properties conferred by the DNA introduced, in a manner similar to that exhibited by their parents.

B.  General Description

The invention provides the leader sequence for the phoA gene in reading frame with a desired heterologous peptide. The phoA promoter and terminator sequences may also be used advantageously, although alternate promoters and positive retroregulators may be substituted which are compatible with bacterial expression. The resulting constructs provide, when transformed into procaryotic hosts, the opportunity to obtain the desired coding sequence in a secreted, biologically active, soluble form free of leader sequence.

This result, yielding secreted protein, is dependent on the peptide being susceptible to processing in conjunction with leader, as set forth above. The amino acid sequence characterizing human growth hormone and tumor necrosis factor are exemplary of susceptible peptide. IL-2 sequences are not susceptible to such processing.

In the approach illustrated below, vectors are provided which contain the phoA promoter, phoA leader sequence and phoA terminator with suitable restriction sites for insertion of the desired coding sequence in reading frame with the leader. The only essential element is the leader sequence; the phoA promoter and terminator are simply convenient in construction. Alternative bacterial promoters such as, for example, the trp promoter or $P_L$ promoter could also be used. Alternative terminators are also available, including, most importantly, the positive retroregulator sequences isolated from B. thuringiensis crystal protein gene, which are also illustrated herein.

C. Standard Methods

Most of the techniques which are used to transform cells, construct vectors, extract messenger RNA, prepare cDNA libraries, and the like are widely practiced in the art, and most practitioners are familiar with the standard resource materials which describe specific conditions and procedures. However, for convenience, the following paragraphs may serve as a guideline.

C.1.    Hosts and Control Sequences

The invention vectors are suitable for procaryotic expression. Procaryotes most frequently are represented by various strains of E. coli, a gram-negative organism. However, other microbial strains may also be used, such as the gram-positive bacilli, for example Bacillus subtilis, various species of Pseudomonas, or other bacterial strains. In such procaryotic systems, plasmid vectors which contain replication sites and control sequences derived from a species compatible with the host are used. For example, E. coli is typically transformed using derivatives of pBR322, a plasmid derived from an E. coli species by Bolivar, et al, Gene (1977) 2:95. pBR322 contains genes for ampicillin and tetracycline resistance, and thus provides additional markers which can be either retained or destroyed in constructing the desired vector. Other commonly used vectors are from the pUC series which contain polylinkers for convenient insertion of desired DNA. Commonly used procaryotic control sequences which include promoters for transcription initiation, optionally with an operator, along with ribosome binding site sequences, are exemplified by such commonly used promoters as the β-lactamase (penicillinase) promoter system (Chang, et al, Nature (1977) 198:1056) and the tryptophan (trp) promoter system (Goeddel, et al Nucleic Acids Res (1980) 8:4057) and the lambda derived P$_L$ promoter and N-gene ribosome binding site (Shimatake, et al, Nature (1981) 292:128), which has been made useful as a portable control cassette, as set forth in published PCT application number WO85/03522 published 15

August, 1985, and assigned to the same assignee. In the instant case, the phoA promoter is illustrated. However, any available promoter system compatible with procaryotes can be used.

## C.2. Transformations

For the procaryotic hosts used to illustrate the invention, the calcium treatment employing calcium chloride, as described by Cohen, S. N., Proc Natl Acad Sci .(USA) (1972) 69:2110, or the RbCl$_2$ method described in Maniatis, et al, Molecular Cloning: A Laboratory Manual (1982) Cold Spring Harbor Press, p. 254 was used.

## C.3. Vector Construction

Construction of suitable vectors containing the desired coding and control sequences employs standard ligation and restriction techniques which are well understood in the art. Isolated plasmids, DNA sequences, or synthesized oligonucleotides are cleaved, tailored, and religated in the form desired.

Site specific DNA cleavage is performed by treating with the suitable restriction enzyme (or enzymes) under conditions which are generally understood in the art, and the particulars of which are specified by the manufacturer of these commercially available restriction enzymes. See, e.g., New England Biolabs, Product Catalog. In general, about 1 μg of plasmid or DNA sequence is cleaved by one unit of enzyme in about 20 μl of buffer solution; in the examples herein, typically, an excess of restriction enzyme is used to insure complete digestion of the DNA substrate. Incubation times of about one hour to two hours at about 37°C are workable, although variations can be tolerated. After each incubation, protein is removed by extraction with phenol/chloroform, and may be followed by ether extraction, and the nucleic acid recovered from aqueous fractions by precipitation with ethanol followed by running over a Sephadex G-50 spin column. If desired, size separation of the cleaved fragments may be performed by polyacylamide gel or agarose gel electrophoresis using standard techniques. A general description of size separations is found in Methods in Enzymology (1980) 65:499-560.

Restriction cleaved fragments may be blunt ended by treating with the large fragment of E. coli DNA polymerase I (Klenow) in the presence of the four deoxynucleotide triphosphates (dNTPs) using incubation times of about 15 to 25 min at 20 to 25°C in 50 mM Tris pH 7.6, 50 mM NaCl, 6 mM $MgCl_2$, 6 mM DTT and 5-10 μM dNTPs. The Klenow fragment fills in at 5' sticky ends but chews back protruding 3' single strands, even though the four dNTPs are present. If desired, selective repair can be performed by supplying only one of the, or selected, dNTPs within the limitations dictated by the nature of the sticky ends. After treatment with Klenow, the mixture is extracted with phenol/chloroform and ethanol precipitated followed by running over a Sephadex G-50 spin column. Treatment under appropriate conditions with S1 nuclease results in hydrolysis of any single-stranded portion.

Synthetic oligonucleotides are prepared by the triester method of Matteucci, et al (J Am Chem Soc (1981) 103:3185) or using commercially available automated oligonucleotide synthesizers. Kinasing of single strands prior to annealing or for labeling is achieved using an excess, e.g., approximately 10 units of polynucleotide kinase to 0.1 nmole substrate in the presence of 50 mM Tris, pH 7.6, 10 mM $MgCl_2$, 5 mM dithiothreitol, 1-2 mM ATP, 1.7 pmoles $\gamma$32P-ATP (2.9 mCi/mmole), 0.1 mM spermidine, 0.1 mM EDTA.

Ligations are performed in 15-30 $\mu$l volumes under the following standard conditions and temperatures: 20 mM Tris-HCl pH 7.5, 10 mM $MgCl_2$, 10 mM DTT, 33 $\mu$g/ml BSA, 10 mM-50 mM NaCl, and either 40 $\mu$M ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 33-100 $\mu$g/ml total DNA concentrations (5-100 nM total end concentration). Intermolecular blunt end ligations (usually employing a 10-30 fold molar excess of linkers) are performed at 1 $\mu$M total ends concentration.

In vector construction employing "vector fragments", the vector fragment is commonly treated with bacterial alkaline phosphatase (BAP) in order to remove the 5' phosphate and prevent religation of the vector. BAP digestions are conducted at pH 8 in approximately 150 mM Tris, in the presence of $Na^+$ and $Mg^{+2}$ using about 1 unit of BAP per g of vector at 60°C for about one hour. In order to recover the nucleic acid fragments, the preparation is extracted with phenol/ chloroform and ethanol precipitated and desalted by application to a Sephadex G-50 spin column. Alternatively, religation can be prevented in vectors which have been double digested by additional restriction enzyme digestion of the unwanted fragments.

### C.4. Probe Hybridization

Probe hybridization is conducted by lysing colonies containing the DNA to be screened and fixing the DNA to nitrocellulose filters by sequential treatment for 5 min with 500 mM NaOH, 1.5 M NaCl, and washing twice for 5 min each time with 5 x standard saline citrate (SSC). Filters are air dried and baked at 80°C for 2 hr. The duplicate filters are prehybridized at 42°C for 6-8 hr with 10 ml per filter of DNA hybridization buffer (5 x SSC, pH 7.0 5x Denhardt's solution (polyvinylpyrrolidine, plus Ficoll and bovine serum albumin; 1 x = 0.02% of each), 50 mM sodium phosphate buffer at pH 7.0, 0.2% SDS, 20 μg/ml poly U, and 50 μg/ml denatured salmon sperm DNA).

The samples are hybridized with kinased probe under conditions which depend on the stringency desired. Typical moderately stringent conditions employ a temperature of 42°C for 24-36 hr with 1-5 ml/filter of DNA hybridization buffer containing probe. For higher stringencies high temperatures and shorter times are employed. The filters are washed four times for 30 min each time at 37°C with 2 x SSC, 0.2% SDS and 50 mM sodium phosphate buffer at pH 7, then are washed twice with 2 x SSC and 0.2% SDS, air dried, and are autoradiographed at -70°C for 2 to 3 days.

## C.5. Site-Specific Mutagenesis

For portions of vectors derived from cDNA or genomic DNA which require sequence modifications, site specific primer directed mutagenesis is used. This is conducted using a primer synthetic oligonucleotide complementary to a single stranded phage DNA to be mutagenized except for limited mismatching, representing the desired mutation. Briefly, the synthetic oligonucleotide is used as a primer to direct synthesis of a strand complementary to the phage, and the resulting double-stranded DNA is transformed into a phage-supporting host bacterium. Cultures of the transformed bacteria are plated in top agar, permitting plaque formation from single cells which harbor the phage.

Theoretically, 50% of the new plaques will contain the phage having, as a single strand, the mutated form; 50% will have the original sequence. The resulting plaques are hybridized with kinased synthetic primer at a temperature which permits hybridization of an exact match, but at which the mismatches with the original strand are sufficient to prevent hybridization. Plaques which hybridize with the probe are then picked, cultured, and the DNA recovered.

In more detail, the DNA fragment containing the sequence to be altered is ligated into an appropriate site of the cloning bacteriophage M13. The ligated phage transduced into competent lysogenic E. coli, such as DG98, and cultured by plating on suitable media. Mini-cultures are screened for recombinant single strand phage DNA containing appropriate inserts and the structure of the desired recombinant phage is confirmed using restriction analysis.

Ten picomoles of the primer oligonucleotide are hybridized to 2.6 μg of ss clone DNA in 15 μl of a mixture containing 100 mM NaCl, 20 mM Tris-HCl, pH 7.9, 20 mM $MgCl_2$ and 20 mM ß-mercaptoethanol, by heating at 67°C for 5 min and 42°C for 25 min. The annealed mixtures are chilled on ice and then adjusted to a final volume of 25 1 of a reaction mixture containing 0.5 mM of each dNTP, 17 mM Tris-HCl, pH 7.9, 17 mM $MgCl_2$, 83 mM NaCl, 17 mM ß-mercaptoethanol, 5 units of DNA polymerase I Klenow fragment, incubated at 37°C for 1 hr. The reactions are terminated by heating to 80°C and the reaction mixtures used to transform competent E.

coli cells, plated onto agar plates and incubated overnight to obtain phage plaques.

Plates containing mutagenized plaques as well as control plates containing unmutagenized phage plaques, are chilled to 4°C and phage plaques from each plate are transferred onto 2 nitrocellulose filter circles by layering a dry filter on the agar plate for 5 min for the first filter and 15 min for the second filter. The filters are then placed on thick filter papers soaked in 0.2 N NaOH, 1.5 M NaCl and 0.2% Triton X-100 for 5 min, and neutralized by layering onto filter papers soaked with 0.5 M Tris-HCl, pH 7.5, and 1.5 M NaCl for another 5 min. The filters are washed in a similar fashion twice on filters soaked in 2 x SSC, dried and then baked in a vacuum oven at 80°C for 2 hr. The duplicate filters are prehybridized at 42°C for 4 hr with 10 ml per filter of DNA hybridization buffer (5 x SSC, pH 7.0, 4 x Denhardt's solution (polyvinylpyrrolidine, ficoll and bovin serum albumin, 1x = 0.02% of each), 0.1% SDS, 50 mM sodium phosphate buffer, pH 7.0 and 100 μg/ml of denatured salmon sperm DNA. $^{32}$P-labeled probes are prepared by kinasing the primer with labeled ATP. The filters are hybridized to 5 x $10^6$ cpm/ml of $^{32}$P-labeled primer in 1-5 ml per filter of DNA hybridization buffer at 64°C for 8 hr.

The filters are washed once at room temperature for 10 min in 0.1% SDS, 20 mM sodium phosphate (buffer) and 6 x SSC; once at 37°C for 20 min in buffer and 2 x SSC; once at 50°C for 20 min in buffer and 2 x SSC; and finally at 60°C for 20 min in buffer and 1 x SSC. The filters are air dried and autoradiographed at -70°C for 4 hr. The desired mutagenized colonies are picked and inocculated into a competent E. coli culture to obtain quantities of the modified DNA. From these cultures, ssDNA is prepared from the supernatant and dsRF-DNA is prepared from the pellet.

### C.6. Verification of Construction

Correct ligations for plasmid construction are confirmed by first transforming a suitable E. coli such as strain MM294 obtained from E. coli Genetic Stock Center, CGSC #6135, or other suitable host with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D. B., et al, Proc Natl Acad Sci (USA) (1969) 62:1159, optionally following chloramphenicol amplification (Clewell, D. B., J Bacteriol (1972) 110:667). The isolated DNA is analyzed by restriction and/or sequenced by the dideoxy method of Sanger, F., et al, Proc Natl Acad Sci (USA) (1977) 74:5463 as further described by Messing, et al, Nucleic Acids Res (1981) 9:309, or by the method of Maxam, et al, Methods in Enzymology (1980) 65:499.

### C.6. Hosts Exemplified

Host strains used in cloning and expression herein are as follows:

For cloning and sequencing, and for expression of construction under control of most bacterial promoters, E. coli strain MM294 (supra), Talmadge, K., et al, Gene (1980) 12:235; Meselson, M., et al, Nature (1968) 217:1110, was used as the host. For expression under control of the $P_L N_{RBS}$ promoter, E. coli strain K12 MC1000 lambda lysogen, $N_7 N_{53} cI857 SusP_{80}$. ATCC

39531 (hereinafter sometimes referred to as MC1000-39531) is used.

For M13 phage recombinants, E. coli strains susceptible to phage infection, such as E. coli K12 strain DG98 are employed. The DG98 strain has been deposited with ATCC and has accession number 39768.

For constructions employing inserts into pUC vectors, E. coli DG99 was used. This strain is analogous to E. coli JM103 or JM105, and contains a lacZVm15 lesion to complement the pUC plasmid, which contains a functional lacZ gene. Thus, desired cultures containing inserts in the pUC vectors grown on X-Gal media will produce white colonies, while blue colonies result in cultures transformed with unaltered pUC vectors.

D. Examples

The following examples are intended to illustrate the invention. Constructions of typical expression vectors for both susceptible and non-susceptible peptide precursor proteins are illustrated, along with results of transformation with these vectors.

D.1. Construction of Source Vectors for phoA Gene Components/Host Expression Vectors

pSYC1015/pSYC997

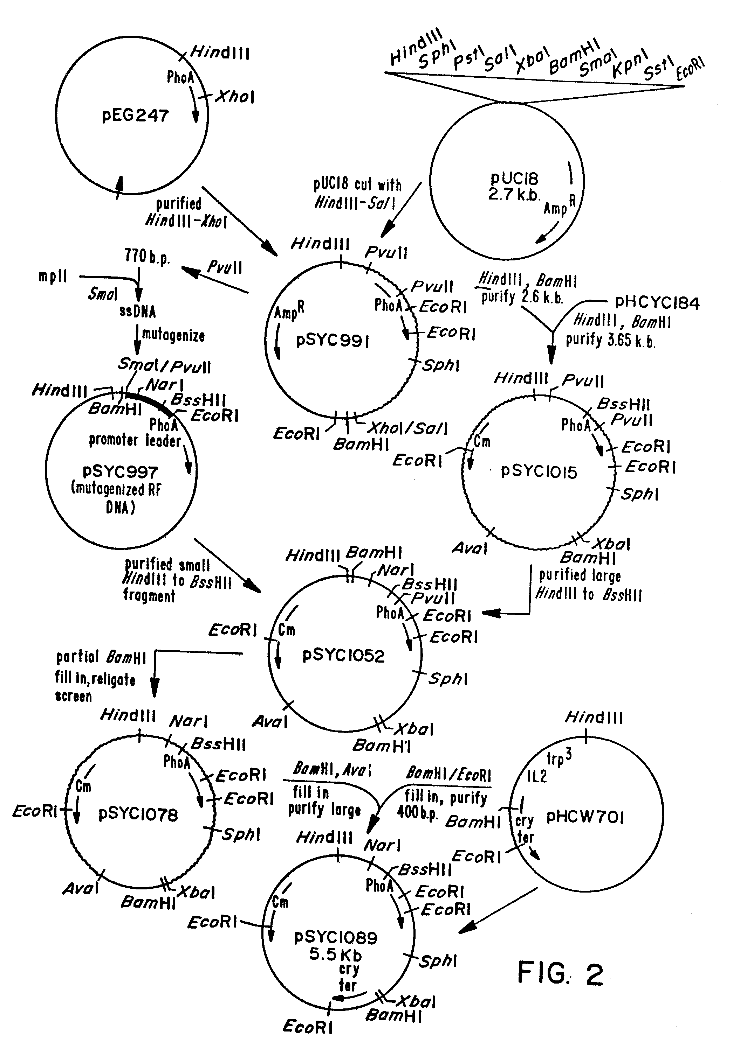

pSYC1015 is an approximately 6.3 kb vector containing the entire phoA gene. pSYC997 contains the phoA gene with a modification to provide a NarI site at the C-terminal end of the leader sequence. The construction of these plasmids, which were used in further vector construction is shown in Figure 2.

Plasmid pEG247 a 25 kb plasmid containing the 2.6 kb phoA structural gene as a HindIII/XhoI fragment was used as a source of the gene. This plasmid was obtained from M. Casadaban and was constructed in a manner analogous to that set forth in Groisman, E. A., et al, Proc Natl Acad Sci (USA) (1984) 81:1840-1843. Indeed, by applying the procedures set forth in the foregoing reference, the phoA gene may be conveniently cloned into any desirable backbone vector.

The HindIII/XhoI 2.6 kb fragment from pEG247 was purified and cloned into pUC18, a 2.7 kb plasmid containing an ampicillin resistance marker and a polylinker permitting convenient insertion of desired sequences. pUC18 was digested with HindIII/SalI, and the linear vector ligated with the isolated phoA fragment. The ligation mixture was used to transform E. coli DG99 to Amp$^R$, and the construction of the intermediate plasmid pSYC991 in successful transformants screened for inserts by detection of white colonies on X-Gal medium was verified. pSYC991 was used both for the construction of pSYC1015 and of pSYC997.

For construction of pSYC1015, pSYC991 was digested with HindIII/BamHI and the approximately 2.6 kb fragment again purified and ligated with the purified 3.65 kb vector fragment from HindIII/BamHI digested pACYC184. pACYC184 is available from ATCC and contains the chloramphenicol resistance gene (Cm$^R$), a bacterial replicon, and HindIII and BamHI sites in the tetracycline resistance gene. The ligation mixture was used to transform E. coli MM294 to Cm$^R$ and the construction of pSYC1015 verified by restriction analysis and sequencing.

pSYC997 was prepared from pSYC991 by site-directed mutagenesis. The PvuII/PvuII 770 base pair fragment was obtained from pSYC991. It includes a portion of the phoA promoter and the upstream N-terminal sequences of the mature alkaline phosphatase, and thus, also, the entire leader sequence. This fragment was ligated into the SmaI site of M13mp11 and single stranded phage was prepared as template for the mutagenesis. In the mutagenesis, the synthetic 26-mer, 5'-TTCTGGTGTCGGCGCCTTTGTCACAG-3' was used as primer and probe. The mutagenized phage particles were then used to prepare RF-DNA as a source for the desired leader sequence containing the NarI site.

### pSYC1089

pSYC1089 contains the modified phoA leader, the phoA coding sequences, and the positive retroregulator of the B. thuringiensis crystal protein gene.

Two additional intermediate plasmids, pSYC1052 and pSYC1078, were constructed, as shown in Figure 2, in order to provide a suitable host vector for the B. thuringiensis positive retroregulator.

pSYC1052 was constructed by ligating the purified small HindIII/BssHII fragment containing the phoA promoter and NarI site from modified leader pSYC997 into HindIII/BssHII-digested pSYC1015, which has, thus, the unmodified phoA sequences deleted. The resulting vector pSYC1052 was confirmed in E. coli transformants to Cm$^R$.

pSYC1078 is a modified form of pSYC1052 with the BamHI site in front of the phoA promoter deleted. In order to delete this BamHI site, pSYC1052 was subjected to partial BamHI digestion, filled in using DNA polymerase I (Klenow) in the presence of the four dNTPs, and religated under blunt-end conditions. The desired resulting plasmid, now containing a unique BamHI site just 3' of the phoA gene, was confirmed after screening successful Cm$^R$ transformants.

The ability of the 3' sequences of the gene encoding crystal protein from B. thuringiensis (the cry gene) to enhance the expression of upstream coding sequences was described and claimed in copending U.S. Patent Application 646,584, filed 31 August 1984, assigned to the same assignee, and incorporated herein by reference. Briefly, these sequences are characterized by a DNA sequence which transcribes to a corresponding RNA transcript capable of forming a stem and loop structure having a cytosine-guanine residue content of about 43%. When ligated about 30-300 nucleotides from the 3' end of the gene, a positive retroregulatory effect is shown on the gene expression. The positive retroregulator was prepared as a 400 bp EcoRI/BamHI restriction fragment, which was blunt-ended and ligated into pLW1, an expression vector for interleukin-2 to obtain pHCW701, which was used as a source for the desired fragment.

pHCW701 was deposited with ATCC under the terms of the Budapest Treaty and has accession no. 39757.

To complete pSYC1089, pHCW701 was digested with EcoRI, filled in using Klenow and the four dNTPs, then digested with BamHI, and the 400 bp fragment containing the positive retroregulator recovered. pSYC1078 was digested with AvaI, filled in with Klenow and the four dNTPs, and then digested with BamHI. The ligation mixture was transformed into E. coli MM294 and the construction of the desired plasmid pSYC1089, a 5.5 kb plasmid conferring Cm$^R$, was confirmed. pSYC1089 contains the sequences for the phoA promoter and leader (with NarI site) sequence and structural gene immediately upstream of a BamHI site, followed by the positive retroregulator sequences of the cry gene.

### D.2. Construction of Expression Vectors for phoA/IL-2 Fusions

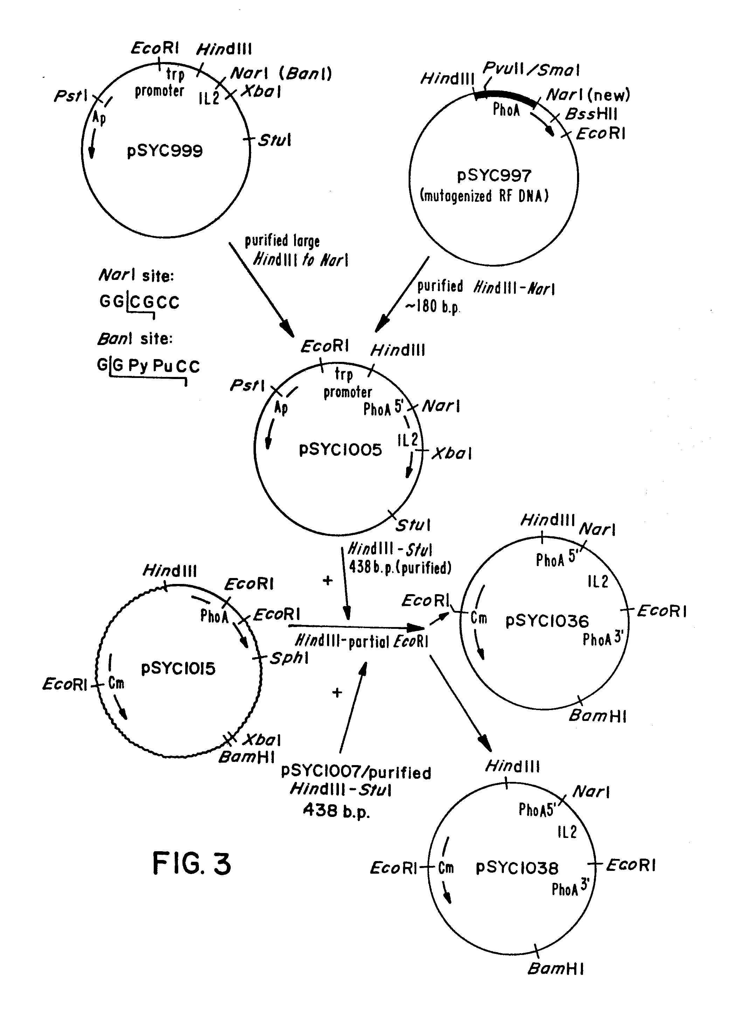

Four expression vectors containing the desired fusion were constructed: pSYC1005 and pSYC1007 (which contain no phoA terminator sequences) and pSYC1036 and pSYC1038 (which do contain phoA terminators). The construction of these vectors is outlined in Figure 3.

pSYC1005 and pSYC1007 differ only in the junction region between IL-2 and the leader sequence; pSYC1007 contains an additional alanine residue at the N-terminus.

The IL-2 coding sequences are obtained, in both of these vectors, from pSYC999, which contains the IL-2 coding sequence under trp promoter control. pSYC999 was prepared from another IL-2-containing plasmid, pLW34, by deleting a BanII/PvuII fragment. pLW34, in turn, was prepared from pLW1, which contains the IL-2 sequence and which was described in U.S. Patent Application Serial No. 646,584 (supra), and deposited with ATCC, accession no. 39405. The conversion of pLW1 to pLW34 is described in an addendum as ¶D.6 below.

pSYC1005 was constructed by ligating the purified 180 bp HindIII/NarI fragment from pSYC997 (which fragment contains the portion of the leader sequence between the new NarI site upstream to the PvuII restriction site, preceded by a short segment of phage DNA) into the large vector fragment purified from HindIII/NarI digested pSYC999. The ligation mixture was transformed into E. coli to Amp$^R$, and the construction of pSYC1005 confirmed by restriction analysis and sequencing. The sequence in the junction region is a straightforward product of NarI sticky end ligation regenerating the alanine codon in position 1 of the IL-2 sequence.

For pSYC1007, pSYC997 was digested with NarI, filled in using DNA polymerase I (Klenow) and then with HindIII. Two fragments from pSYC999 were then used: the large HindIII/XbaI fragment containing most of the vector up through a portion of the coding sequence of IL-2, and the short fragment spanning the NarI/XbaI portion of the IL-2 coding sequence obtained by cleaving with BanI, filling in with DNA polymerase I, and then with XbaI. The three-way ligation between the pSYC999 vector and the short fragments containing filled NarI sites results in the fusion sequence 5'-AAGGCGGCGCCT-3' encoding Lys-Ala-Ala-Pro — i.e., it includes an additional alanine residue at the N-terminus of the IL-2. The correct construction of this vector, pSYC1007, was also confirmed.

pSYC1005 and pSYC1007 were transformed into E. coli MM294, and the transformants were grown under conditions similar to those described by Inouye, H., et al, J Bact (1981) 146:668-675. Briefly, the cells were grown in 10 mM $KH_2PO_4$ (MOPS medium) for 6-8 hr and then induced by washing the cells and resuspending in MOPS medium containing 0.1 mM $KH_2PO_4$ for 16-24 hr, as described by Neihardt, F.C., et al, J Bact (9174) 119:736-747. Osmotic shockates of the culture, prepared as described by Nossal, N.G., et al, J Biol Chem (1966) 241:3055-3062, presumably released the material contained in the periplamic space, but not intracellular material. The osmotic shockates gave no activity. Sonicates, however, which release total cellular content, showed IL-2 activity of 2.1 x $10^{-4}$ units/ml for pSYC1005 transformants, and 5.5 x $10^3$ units/ml for

pSYC1007 transformants. Thus, the IL-2 activity produced was not secreted into the periplasm.

Biological activity of recombinant IL-2 in all cases was measured using the murine IL-2-dependent cell line HT-2 and measuring the incorporation of [$^3$H] thymidine, according to the procedure of Watson, J., J Exp Med (1979) 150:1510 and Gillis, S., et al, J Immunol (1978) 120:2027.

Modifications of the foregoing expression vectors were made to insert phoA terminators operably disposed with respect to the coding sequences. The terminators were obtained from pSYC1015 (supra) as the large vector HindIII/partial EcoRI (blunt ended with Klenow and the four dNTPs) digest fragment. As shown in Figure 3, this fragment carries the Cm$^R$ gene, the replicon, and the downstream portion of the phoA gene. The DNAs containing phoA promoter and leader sequence, with the leader sequence in frame with the IL-2 encoding sequence were obtained from either pSYC1005 or pSYC1007, then purified, and ligated into the above-prepared vector fragment of pSYC1015 to obtain pSYC1036 and pSYC1038 respectively. The respective ligation mixtures were transformed into E. coli to Cm$^R$ and correct constructions confirmed.

Transformants from each of pSYC1036 and pSYC1038, grown and induced as described above, produced IL-2 activity at over 100 times the levels obtained for the parent plasmids without terminators. Again, osmotic shockates were inactive, but sonicates of the cell cultures showed 1.2 x 10$^6$ units/ml and 2 x 10$^6$ units/ml of IL-2 activity for pSYC1036 and pSYC1038 extracts respectively.

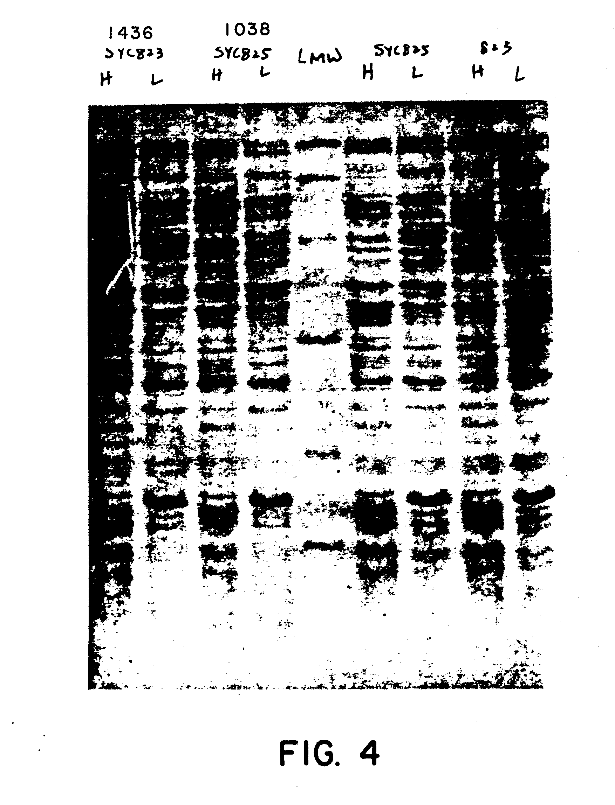

Figure 4 shows the results of SDS-PAGE on sonicates from induced and uninduced cultures of the above transformants. Lanes 1 and 2 are, respectively uninduced and induced pSYC1036 transformants, lanes 3 and 4 uninduced and induced pSYC1038 transformants. Both the induced cultures have bands corresponding to molecular weights of unprocessed "pre" IL-2 which bands are absent in the uninduced cultures.

### D.3. Construction of Expression Vectors for the Susceptible Peptide hGH

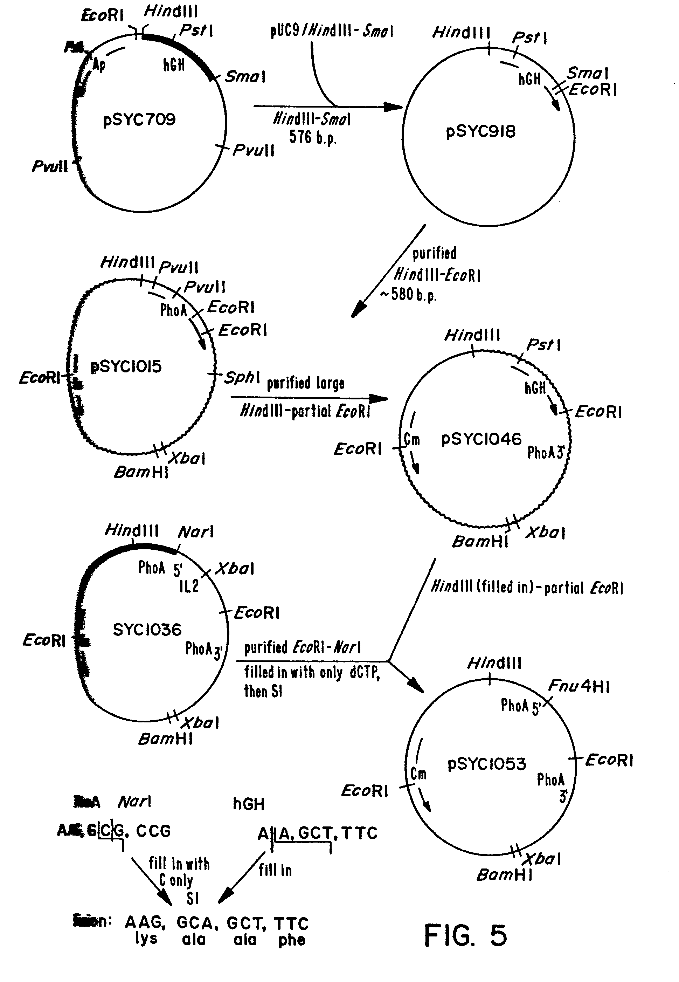

An in-frame fusion with the coding sequence for hGH, but with the fusion containing a codon for an alanine preceding the native N-terminus was constructed in a manner similar to that for IL-2 above. The resulting vector, pSYC1053 contains the in-frame fusion under control of the phoA promoter and terminator. (See Figure 5.)

The hGH coding sequences are obtained from pSYC709 which plasmid was described in U.S. Serial No. 583,472, filed 2 March 1984, assigned to the same assignee and incorporated herein by reference. pSYC709 was deposited with ATCC on 7 February 1984 and has accession number 39602. The coding sequences were obtained as a 576 bp HindIII/SmaI digest from pSYC709 and cloned into HindIII/SmaI cleaved pUC9 to obtain pSYC918 as an intermediate, thus obtaining an EcoRI site immediately downstream of the SmaI site used to obtain the coding sequence. Accordingly, pSYC918 was digested using HindIII/EcoRI to produce a 580 bp fragment, which was purified and cloned into the purified large vector fragment from HindIII/partial EcoRI digested pSYC1015 (supra). The resulting plasmid, pSYC1046, was transformed into E. coli to $Cm^R$ and confirmed to contain the hGH coding sequences in operable linkage to the phoA terminator sequences.

To obtain the desired expression vector, pSYC1053, pSYC1046 was digested with HindIII, filled in with DNA polymeraseI (Klenow) and then partially digested with EcoRI to delete the vector sequences upstream of the N-terminal HindIII site of hGH to the EcoRI site in the Cm$^R$ gene. This vector fragment was then ligated with the phoA promoter/leader sequences and the deleted portion to the EcoRI site of the Cm$^R$ gene by first obtaining a NarI digest of pSYC1036, filling in with dCTP in the presence of Klenow and then blunt ending with S1, followed by digestion with EcoRI and isolation of the purified fragment. The two EcoRI/blunt fragments were then ligated and transformed into E. coli to Cm$^R$ and the construction of the desired plasmid pSYC1053 confirmed. The sequence at the junction of the phoA leader and hGH coding sequences is 5'-AAGGCAGCTTTC-3' which encodes Lys-Ala-Ala-Phe.

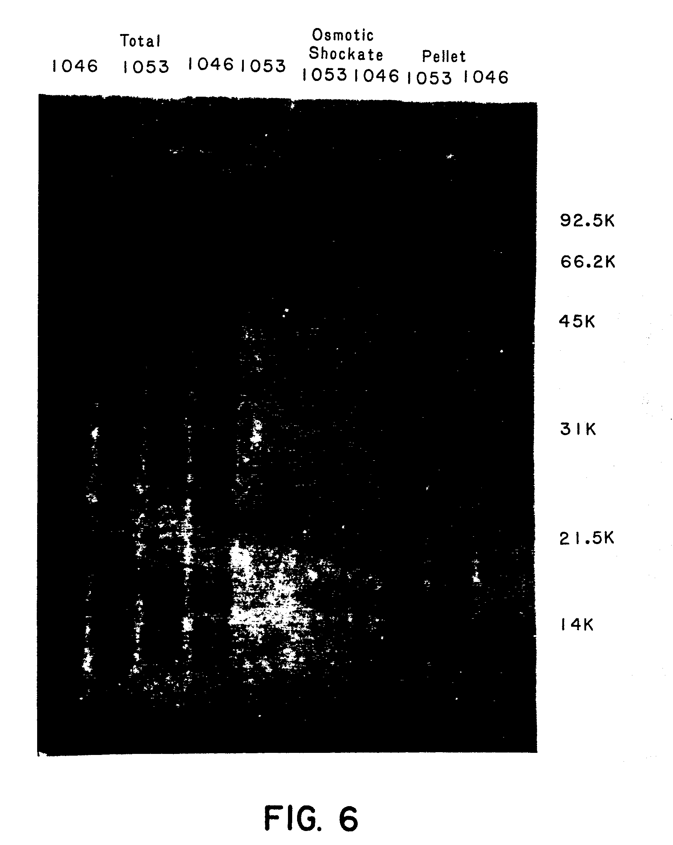

hGH production was assayed in osmotic shockates and sonicates of E. coli MM294 transformed with pSYC1053 and control intermediate plasmids. Cells were transformed, grown and induced in the presence of low phosphate as set forth above. Figure 6 shows the results of SDS-PAGE on whole cell contents (sonicates) and osmotic shockates of these cells. As shown in Figure 6, cells transformed with pSYC1053 have a unique band at the expected size for processed hGH in the osmotic shockate fraction, as well as in the sonicate. Thus, hGH is a susceptible protein, capable of secretion into the periplasmic space with deletion of the signal sequence.

## D.4.  Construction of Expression Vectors for the Susceptible Peptide TNF

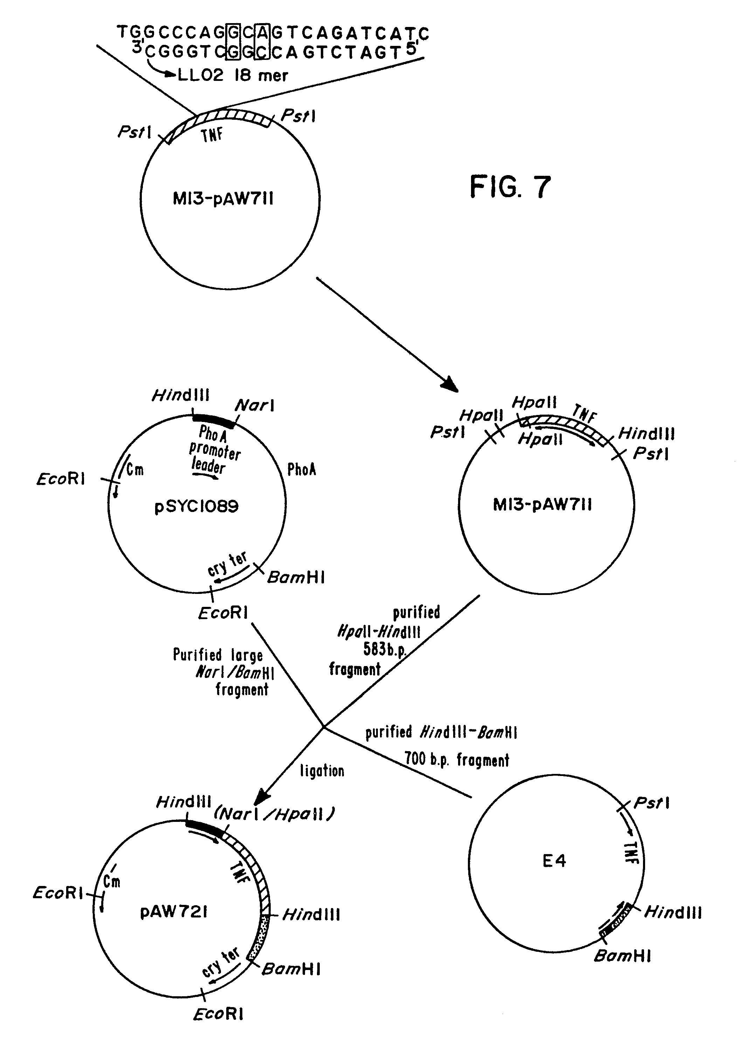

pAW721 is an expression vector which contains the coding sequence for tumor necrosis factor (TNF) in reading frame with the phoA leader under control of the phoA promoter and B. thuringiensis crystal protein (cry) positive retroregulator system. Its construction is shown in Figure 7.

pAW721 is constructed using pSYC1089 as the source of control and leader sequences, and a modified M13-pAW721 and pE4 to furnish the TNF encoding sequence. pE4 and the plasmid which provides the relevant insert in M13-pAW721 are both described in detail in Wang, A. etal, Molecular Cloning of the Complementary DNA for Human Necrosis Factor, 228, 149-154, 4/12/85 and incorporated herein by reference. pE4 was deposited with ATCC 15 October 1984 and has accession no. 39894 pE4 is a cDNA clone prepared by the method of Okayama and Berg, Mol Cell Biol (1983) 3:280, which contains the entire TNF gene as a HindIII cassette.

The modified M13-pAW721 was used to provide the upstream sequences of the gene. It was constructed from pE4 by ligation of a PstI/PstI fragment from pE4 into the PstI site of M13mp18. The sequence in the resulting vector was then altered by site-specific mutagenesis to produce an HpaII site at the upstream end of the gene, using the 18-mer 5'-TGATCTGACCGCCTGGGC-3' as primer. The modified M13 vector was designated M13-pAW721'.

The desired expression vector, pAW721, was obtained in a three-way ligation from a mixture containing:

1)  the purified large vector NarI/BamHI fragment from pSYC1089, which contains the phoA

promoter, phoA leader encoding sequence, Cm$^R$, the appropriate replicon, and the cry positive retroregulator;

2) the purified HpaII/HindIII 584 bp fragment from M13-pAW721', which contains the upstream coding region of the TNF sequence; and

3) the purified 700 bp HindIII/BamHI fragment from pE4, which contains the downstream portion of the TNF structural gene.

The ligation mixture was used to transform E. coli MM294 to Cm$^R$, and the correct construction of pAW721 confirmed by restriction analysis and sequencing.

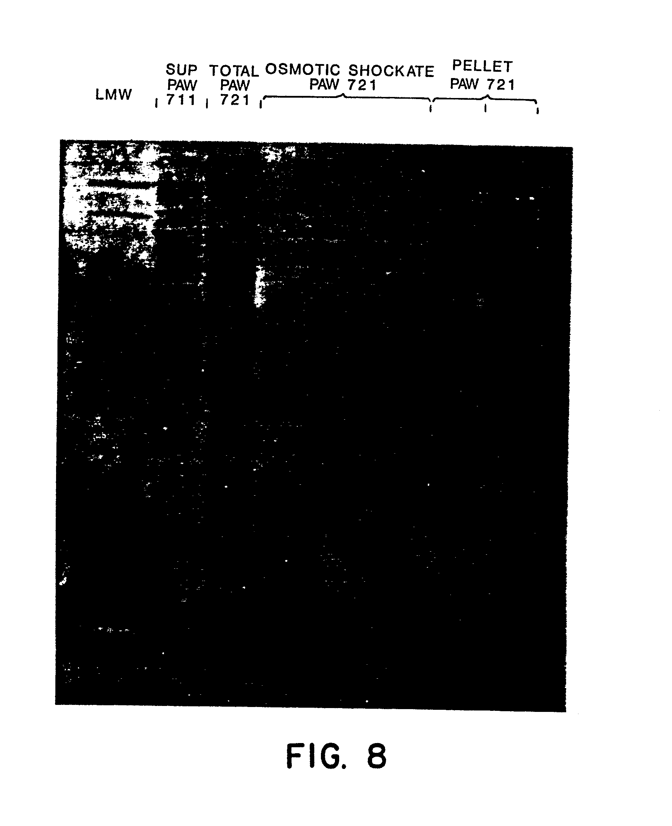

pAW721 transformed E. coli MM294 cells were grown, induced and extracted as described above. Figure 8 shows the results obtained for both osmotic shockate and total sonicate. Lane 1 contains molecular weight markers, and shows the location expected for TNF. Lane 3 shows the total sonicate contains TNF along with a multitude of other proteins but at a lower level in comparison to other proteins than is obtained using pAW711 transformants (lane 2). Lanes 4, 5, and 6 represent various concentrations of the osmotic shockate showing the TNF band in the presence of a limited number of impurities. And lanes 7 and 8 represent the proteins remaining in the pellet from the osmotic shockate. TNF is presumably still present, although as a minor constituent.

The results in Figure 8 show that TNF is a susceptible protein which is secreted, minus the leader sequence, into the periplasm. TNF can thus utilize the pre-sequences of the phoA gene to effect secretion and proper folding.

The activity of the TNF obtained from the pAW721-transformed cells was verified by subjecting the osmotic shockate to the L-929 cytotoxicity assay described in ¶D.5 below. The extracts contained TNF activity.

### D.5. Cytotoxic Assay Procedure

The L-929 assay system is an improved convenient _in vitro_ assay which permits rapid measurement of TNF activity. Its degree of correlation with the _in vivo_ tumor necrosis assay of Carswell is, at present, unknown; however, as it utilizes murine tumor cells specifically, the correlation is expected to be high. The protein designated lymphotoxin in EPO publication no. 0100641 (supra) also gives activity in this assay. The assay is similar in concept to that disclosed in U.S. 4,457,916 which used murine L-M cells and methylene blue staining. however, the L-929 assay has been shown to correlate (for HL-60-derived TNF) with human tumor cell line cytotoxicity (see ¶D.1.b).

In the L-929 assay system herein, L-929 cells are prepared overnight as monolayers in microtiter plates. The test samples are diluted 2-fold across the plate, UV irradiated, and then added onto the prepared cell monolayers. The culture media in the wells are then brought to 1 µg/ml actinomycin D. The plates are allowed to incubate 18 hr at 37°C and the plates are scored visually under the microscope. Each well is given a 25, 50, 75, or 100% mark signifying the extent of cell death in the well. One unit of TNF activity is defined as the reciprocal of the dilution at which 50% killing occurs.

In addition, a more sensitive version of this assay was developed that monitors the release of $^{35}S$ labeled peptides from prelabeled cells, when treated with the test sample and actinomycin D. This version of the assay can be used to quantitate potency, e.g., to evaluate the relative potency of oocyte translated material. Briefly, actively growing L-929 cultures are labeled with $^{35}$S methionine (200 μCi/ml) for 3 hr in methionine-free media supplemented with 2% dialyzed fetal calf serum. The cells are then washed and plated into 96 well plates, incubated overnight, and treated the next day with 2-fold dilutions of test samples and 1 μg/ml actinomycin D. The cultures were then incubated at 37°C for 18 hr. One hundred μl supernatant aliquots from each well were then transferred onto another 96 well plate, acid (TCA) precipitated, and harvested onto glass fiber filters. The filters were washed with 95% ethanol, dried, and counted. An $NP_{40}$ detergent control is included in every assay to measure maximum release of radioactivity from the cells. The percent $^{35}$S release is then calculated by the ratio of the difference in count between the treated cells and untreated controls divided by the difference between $NP_{40}$ treated cells and untreated controls, i.e., by the ratio:

$$\% \text{ release} = \frac{\text{sample} - \text{cell control}}{NP_{40} - \text{cell control}} \times 100.$$

Higher TNF potency results in higher values of this ratio.

## D.6. Construction of pLW34

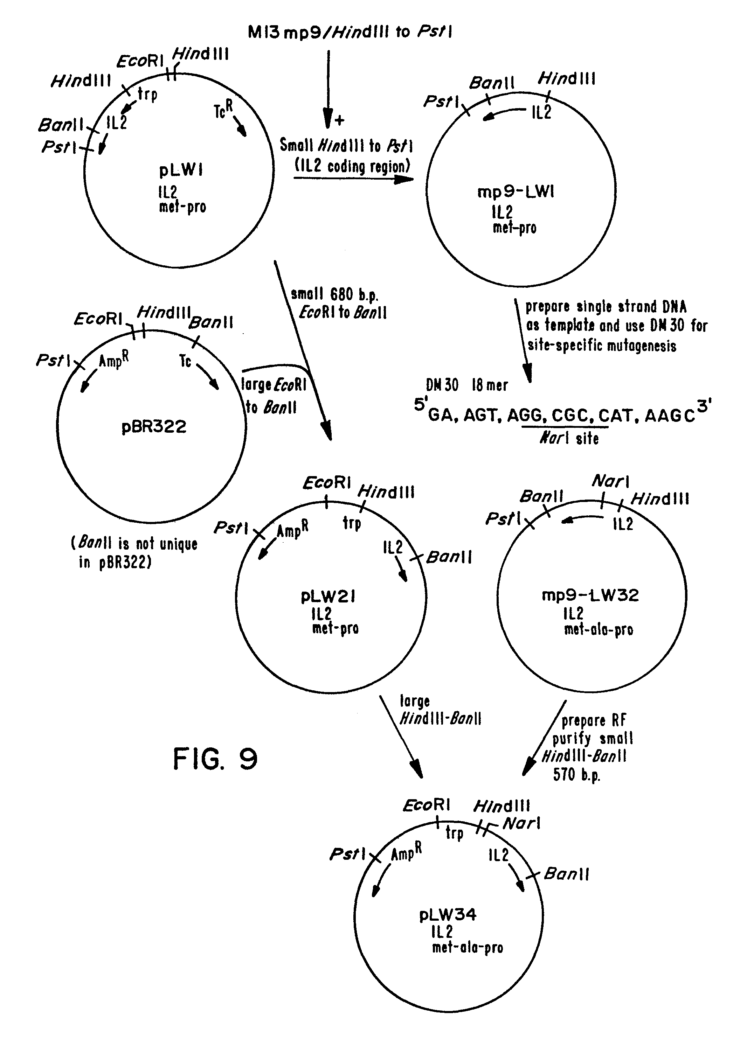

pLW34 is a modified form of pLW1 wherein the IL-2 gene is provided a NarI site at the upstream end and the carrier plasmid is converted from tetracycline resistance to ampicillin resistance. These modifications are shown in Figure 9 and as follows:

pLW1, a Tc[R] plasmid, containing the IL-2 gene in front of the trp promoter was used as a source of the IL-2 coding region for cloning into M13mp9 in order to perform site-specific mutagenesis. pLW1 was digested with HindIII and PstI and the fragment containing the IL-2 coding region was inserted into HindIII/PstI digested M13mp9. The resulting M13 vector was subjected to site-specific mutagenesis using the synthetic 18-mer: 5'-GAAGTAGGCGCCATAAGC-3', which provides a NarI site and an additional Ala codon at the start of the IL-2 gene, as primer. The resulting mutant vector M13-LW32 was converted to the replicative form and digested with HindIII and BanII to provide the 570 bp fragment containing the modified portion of the gene.

To provide the vector portion, control sequences, and remaining portion of the IL-2 sequence, pLW1 was digested with EcoRI and BanII and the 680 bp fragment containing the trp promoter and upstream portion of the gene isolated and ligated into EcoRI/BanII (partial) large vector fragment obtained from pBR322. The resulting plasmid, pLW21 which contains the Amp[R] gene but unmodified IL-2 was then digested with HindIII and BanII and ligated to the HindIII/BanII fragment obtained from M13-LW32 containing the modified IL-2 gene upstream portions. The resulting vector, pLW34 contains a unique NarI site at the start of the IL-2 gene which encodes the starting sequence Met-Ala-Pro.

Applicants have deposited with the American Type Culture Collection, Rockville, MD, USA (ATCC) the following plasmids in host organisms. Deposits were made on the indicated dates and the deposits were assigned the ATCC accession nos. shown. These deposits were made under the provisions of the Budapest Treaty on the International Recognition of the Deposit of Microorganisms for the Purposes of Patent Procedure and the Regulations thereunder (Budapest Treaty). This assures maintenance of a viable culture for 30 years from date of deposit. The organisms will be made available by ATCC under the terms of the Budapest Treaty, and subject to an agreement between Applicants and ATCC which assures unrestricted availability upon issuance of the pertinent US patent. Availability of the deposited strain is not to be construed as a license to practice the invention in contravention of the rights granted under the authority of any government in accordance with its patent laws.

| Plasmid | CMCC | Date of Dep. | ATCC No. |
|---------|------|--------------|----------|
| pSYC1053/E. coli | 2198 | 17 May 1985 | 53131 |
| pAW721/E. coli | 2190 | 17 May 1985 | 53132 |

CLAIMS:

1. A recombinant DNA sequence useful in expression and secretion of a desired coding sequence in procaryotic cells which sequence encodes a fusion protein having as its N-terminal portion the leader sequence of alkaline phosphatase A (phoA) and as its C-terminal portion the desired peptide.

2. A DNA sequence as claimed in claim 1, wherein the desired protein is a protein susceptible to processing.

3. A DNA sequence as claimed in claim 1 or claim 2, wherein the desired protein is hGH or TNF..

4. A DNA sequence as claimed in claim 3 which is pSYC1053 or pAW721.

5. A DNA sequence as claimed in any one of claims 1 to 3 which further includes, in operable linkage with said DNA sequence, a promotor compatible with procaryotic hosts.

6. A DNA sequence as claimed in claim 5, wherein the promotor is the phoA promotor.

7. A DNA sequence as claimed in any one of claims 1 to 3, 5 or 6 which further includes, in operable linkage with said DNA sequence, a bacterially compatible terminator sequence.

8. A DNA sequence as claimed in claim 7, wherein the terminator is the phoA terminator or the terminator for the B. thuringiensis crystal protein gene (cry).

9. A DNA sequence as claimed in any one of claims 5 to 8 which is disposed on a bacterial cloning vector.

10. Recombinant host cells transformed with the vector of claim 9.

11. The use of the leader sequence of alkaline phosphatase A (phoA) as the N-terminal portion of a recombinant DNA sequence when linked to a desired coding sequence as the C-terminal portion of such recombinant DNA sequence in the expression of said desired coding sequence and secretion of the corresponding peptide.

12. The use claimed in claim 11 when said recombinant DNA sequence is further defined by the specific feature of any one of claims 2 to 9.

13. A method for secreting a desired soluble biologically active protein which method comprises culturing cells as defined in claim 10.

14. A method as claimed in claim 13, wherein the protein is hGH or TNF.

AAGCCTTTCGACATTATCGTCACTCCAATGCTTCGCAATATGCCCCAAAATGACCAACAGCCGGTTGATTCATCAGGTAGAGGCGGCCGCTGTACGAGGTAAAC
TCGAAAGCCTCTAATAGCAGTGACGTTACGAAGCGGTTATACCGGCGTTTTACTGGTTGTCGCCAACTAACTAGTCCATCTCCCCCGGCGACATGCTCCATTTC
AlaLeuGlnIleIleValThrAlaMetLeuArgAsnMetAlaGlnAsnAspGlnGlnArgLeuIleAspGlnValGluGlyAlaLeuTyrGluValLys

CCCGATGCCAGCATTCCTGACGACCATACGGACCTGCTGCCGCGATTACGTAAAGAAGTTATTGAAGCATCCTCGTCAGTAAAAAGTTAATCTTTTCAACA
GGGCTACGGTCGTAAGGACTGCTGCTATGCCTCGACGACGCGCTAATGCATTTCTTCAATAACTTCGTAGGAGCAGTCATTTTTCAATTAGAAAGTTGT
ProAspAlaSerIleProAspAspAspThrGluLeuLeuArgAspTyrValLysLysLeuLeuLysHisProArgGln

GCTGTCATAAAGTTGTCACGGCCGAGACTTATAGTCGCTTTGTTTTTATTTTTTAATGTATTTGTACATGGAGAAAATAAAGTGAAACAAAGCACTATTG
CGACAGTATTTCAACAGTGCCGGCTCTGAATATCAGCGAAACAAAAATAAAAAATTACATAAACATGTACCTCTTTTATTTCACTTTGTTTCGTGATAAC
                        PB                                          SD        MetLysGlnSerThrIle
                                                                                      -20

CACTGCCACTCTTACCGTTACTGTTTACCCCTCTGACAAAACCCCCGACACCAGAAATGCCTGTTCTGGAAAACCGGGCTGCTCAGGGCCGATATTACTGC
GTGACCGTGAGAATGGCAATCACAAATGGGGACACTGTTTTCGGGCCTCGTGGTCTTTACCGACAAGACCTTTTGGCCCCGACGAGTCCCGCTATAATGACC
AlaLeuAlaLeuLeuProLeuLeuPheThrProValThrLysAlaArgThrProGluMetProValLeuGluAsnArgAlaLaGlnGlyAspIleThrAla
          -10                           -1  1' +1                              10

ACCCGGCGGTGCTCGCCCGTTTAACCGGTGATCAGACTGCCCGCTCTGCGTGATTCTCTTACCGATAAACCTGCAAAAAAATATTATTTTGCTGATTGGCGAT
TGGGCCGCCACGAGCGGCAAATTGCCCACTAGTCTGACGGGCGAGACGCACTAAGAGAATGCCTATTTGGACGTTTTTTTATAATAAAACGACTAACCGCTA
ProGlyGlyAlaArgArgLeuThrGlyAspGlnThrAlaAlaLeuArgAspSerLeuSerAspLysProAlaLysAsnIleIleLeuLeuIleGlyAsp
     20                  30                    40                      50

GGGATGGCGGACTGGGGATCC
CCCTACCCCCTGACCCCTAG
GlyMetGlyAsp

FIG. I

FIG. 2

FIG. 3

FIG. 4

FIG. 5

6(9)

0196864

FIG. 6

FIG. 7

FIG. 8

FIG. 9